# EUROPEAN PATENT APPLICATION

(11) **EP 2 628 482 A1**
(43) Date of publication of application: **21.08.2013**
(21) Application number: 12075016.1
(22) Date of filing: 17.02.2012
(51) Int. Cl.: A61K 31/44, A61K 31/551, A61P 35/00

(54) **Rho kinase inhiitors for use in the treatment of neuroblastoma**

(71) Applicant: Academisch Medisch Centrum, 1105 AZ Amsterdam (NL)
(72) Inventor: Versteeg, Rogier, 1012 TC Amsterdam (NL); Koster, Jan Johammes Bernardus, 2135 SH Hoofddorp (NL); Molenaar, Jan Jasper, 1187 TP Amstelveen (NL); Westerhout, Ellen, 1068 ZX Amsterdam (NL)

(57) **Abstract**

The invention therefore provides a Rho kinase inhibitor for the treatment of neuroblastoma. Preferably, said Rho kinase inhibitor causes differentiation of neuroblastoma cells. In a preferred embodiment, said Rho kinase inhibitor is selected from the group consisting of (+)-(R)-trans-4-(1-aminoethyl)-N-(4-piridyl)cyclohexanecarboxamide, 4[(1R)-1-aminoethyl]-N-(4-piridyl)benzamide (Fasudil), 1-(1-Hydroxy-5-isoquinolinesulfonyl)homopiperazine] (Hydroxyfasudil), 2-[4-(1H-indazol-5-yl)phenyl]-2-propanamine, N-(3-methoxybenzyl)-4-(4-piridyl)benzamide, 4-[(trans-4-aminocyclohexyl)amino]-2,5-difluorobenzamide, 4-[(trans-4-aminocyclohexyl)amino]-5-chloro-2-fluorobenzamide, 5-(hexahydro-1H-1,4-diazepin-1-ylsulfonyl)isoquinoline, Isoquinolinesulfonamide (K-115), (S)-(+)-2-methyl-1-[(4-methyl-5-isoquinoline)sulfonyl]-homopiperazine (H-1152P), N-(4-Pyridyl)-N'-(2,4,6-trichlorophenyl)urea, Y-27632, Y-39983, and ROCK siRNA or a pharmaceutically acceptable salt thereof.

## Description

### TECHNICAL FIELD OF THE INVENTION

The present invention relates to Rho kinase inhibitors for the treatment of neuroblastoma in a mammal.

### BACKGROUND OF THE INVENTION

Neuroblastoma is a childhood tumour of the peripheral sympathetic nervous system. Neuroblastoma is a solid tumor and commonly occurs in infants and children younger than 5 years, though it may rarely occur in older children and adults. Most neuroblastoma start in and around the adrenal glands. Neuroblastoma may also begin in the abdomen, and in nerve tissue in the neck, chest, and pelvis, where nerve cells are present. The cancer often spreads to other parts of the body, such as the lymph nodes, bones, bone marrow, eyes, liver, skin and the tissue that surrounds the spinal cord. The pathogenesis has for a long time been quite enigmatic, as only very few gene defects were identified in this often lethal tumour. Frequently detected gene alterations are limited to MYCN amplification (20%) and ALK activations (7%).

Neuroblastoma is the second most common solid tumor in childhood. In the advanced stage of the disease, treatment of neuroblastoma is successful in less than a half of patients. The effective treatment of neuroblastoma, either at advanced stage or earlier stage of minimal residual disease, remains indeed one of the major challenges in pediatric oncology. The 5 year survival for the metastatic disease is still less than 60% and, consequently, novel therapeutic approaches are needed.

Neuroblastoma has a highly variable clinical outcome, with an excellent prognosis for stage 1 and 2 tumours, but a poor outcome for high stage tumours. Stage 4S neuroblastoma are metastasized but nevertheless undergo spontaneous regression. Low stage tumours are marked by numeric changes of chromosomal copy numbers, while high stage tumours typically show structural chromosomal defects resulting in e.g. hemizygous deletions of the chromosomal regions 1p36 or 11 q and gain of 17q. Age at diagnosis above 1.5 year is associated with high stage tumours and poor outcome.

At present, treatment of neuroblastoma typically employs a combination of the standard anticancer agents, such as cyclophosphamide (ifosfamide), cisplatin (carboplatin), vincristine, doxorubicin, etoposide, teniposide, topotecan and melphalan. Unfortunately, neuroblastoma is commonly resistant to such conventional anticancer agents, and the cancer relapses after completion of treatment. Thus, there remains a longstanding need for alternative treatments for neuroblastoma. The present invention addresses this need.

### SUMMARY OF THE INVENTION

The invention is based on the observation that defects in genes functioning in neuritogenesis are associated with aggressive stage of neuroblastoma. The inventors have identified by whole genome sequencing of 87 neuroblastoma tumors that genes functioning in neurite growth cone biology and in neuritogenesis are frequently affected by mutations of their coding DNA sequences. Among them, a series of genes function in the Rac/Rho pathway. Rac signalling stimulates neuritogenesis, while Rho signalling is inhibitory. Rac as well as Rho proteins are inhibited by GEFs (guanine nucleotide exchange factor) and activated by GAPs (GTPase activating proteins). The neuroblastoma series showed predominantly mutations in GAPs specific for Rho and GEFs specific for Rac.. These mutations are almost all found in high stage neuroblastoma. Without wishing to be bound by theory, the inventors have the opinion that the Rac-Rho signalling is out of balance and results in inhibition of neuritogenesis. The inventors therefore believe that restoration of this balance will therefore stimulate neuritogenesis, induce differentiation of neuroblastoma cells and therefore constitutes a means to inhibit progression of neuroblastoma.

These findings of mutations in genes tipping the balance in Rac/Rho regulation of neuritogenesis towards inhibition of neuritogenesis and therefore neuroblastoma pathogenesis, suggest that inhibitors of ROCK can stimulate neuritogenesis and thereby counteract proliferation of neuroblastoma cells.

To test this idea, the inventors have treated 6 neuroblastoma cell lines with the ROCK inhibitors Y27632 and HA1077. This resulted in inhibition of proliferation of all 6 cell lines as tested for Y27632 and HA1077 with an MTT assay, morphological differentiation and generation of neurites to a variable degree as shown by light microscopy and a change in actin skeleton organization as shown by phalloidin staining and fluorescence microscopy in 2 of the 2 tested cell lines.

From this the inventors conclude that (i) neuroblastoma tumors frequently have defects in genes functioning in neuritogenesis; (ii) impaired neuritogenesis contributes to neuroblastoma pathogenesis; (iii) treatment of neuroblastoma cell lines with ROCK inhibitors stimulates neuritogenesis and differentiation and inhibit proliferation; (iv) ROCK inhibitors can be clinically used to treat neuroblastoma patients

The invention therefore provides a Rho kinase inhibitor for the treatment of neuroblastoma. Preferably, said Rho kinase inhibitor causes differentiation of neuroblastoma. In a preferred embodiment, said Rho kinase inhibitor is selected from the group consisting of:
- (+)-(R)-trans-4-(1-aminoethyl)-N-(4-piridyl)cyclohexanecarboxamide,
- 4[(1R)-1-aminoethyl]-N-(4-piridyl)benzamide (Fasudil),
- 1-(1-Hydroxy-5-isoquinolinesulfonyl)homopiperazine] (Hydroxyfasudil)
- 2-[4-(1H-indazol-5-yl)phenyl]-2-propanamine,
- N-(3-methoxybenzyl)-4-(4-piridyl)benzamide,
- 4-[(trans-4-aminocyclohexyl)amino]-2,5-difluorobenzamide,
- 4-[(trans-4-aminocyclohexyl)amino]-5-chloro-2-fluorobenzamide,
- 5-(hexahydro-1H-1,4-diazepin-1-ylsulfonyl)isoquinoline,
- Isoquinolinesulfonamide (K-115),
- (S)-(+)-2-methyl-1-[(4-methyl-5-isoquinoline)sulfonyl]-homopiperazine (H-1152P),
- N-(4-Pyridyl)-N'-(2,4,6-trichlorophenyl)urea,
- Y-27632,
- Y-39983, and
- ROCK siRNA,
or a pharmaceutically acceptable salt thereof.

The invention further provides a pharmaceutical composition for the treatment of neuroblastoma, comprising as active compound a therapeutically effective amount of the Rho kinase inhibitor as defined above and a pharmaceutically acceptable carrier. Preferably, said pharmaceutical composition further comprises a further medicament having anti-neuroblastoma activity.

Furthermore, the invention provides a kit comprising the pharmaceutical composition as defined above and a further medicament having anti-neuroblastoma activity.

In a preferred embodiment of said pharmaceutical composition of the invention or kit of the invention, said further medicament is selected from the group consisting of:
a) a differentiation agent;
b) an immunotherapeutic agent directed against neuroblastoma cells;
c) a chemotherapy agent
for simultaneous, separate or sequential use for the treatment of neuroblastoma.

The invention further provides a method for treating a patient suffering from neuroblastoma which comprises administering a therapeutically effective dosis of Rho kinase inhibitor as defined above, the pharmaceutical composition as defined above, or the kit as defined above.

### BRIEF DESCRIPTION OF THE FIGURES

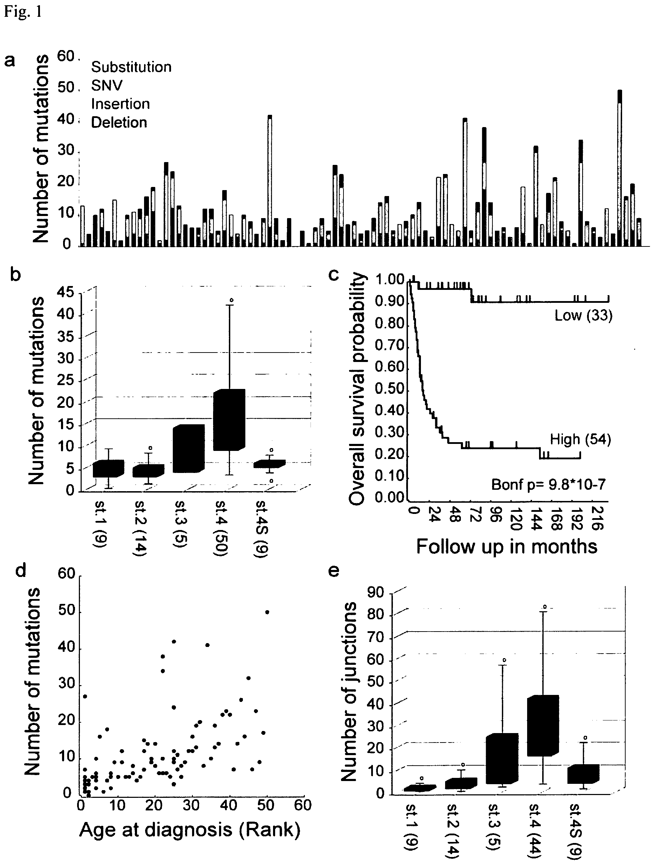

**Figure 1** **(A-E) shows the frequency of amino acid changing somatic mutations in neuroblastoma correlates with age, stage and survival. a,** The number of amino acid changing mutations in 87 primary neuroblastoma (Single Nucleotide Variants (SNVs). Grey shades in the bars indicate from under to top: deletions, insertions, snv's, and substitutions. Numbers shown are events after CGAtools CallDiff with somatic scores >0.1 and not present in dbSNP130, nor in 46 reference genomes released by Complete Genomics. **b**, Average number of mutations per tumour stage (INSS, stage 1: n=9,, stage 2, n=14, stage 3, n=5, stage 4, n=50 and stage 4S, n=9). Boxes include 50% of data and error bars indicate extremes with a maximum of 2 times the box size. **c**, Kaplan Meier curves for tumours with high versus low frequency of mutations. The optimal cut off level for the categories was determined by Kaplan scanning (see supplementary materials and methods). The significance (logrank test) was corrected for the multiple testing (Bonferoni correction)('bonfp'). Number of patients per group is shown between brackets. **d**, Age at diagnosis (rank-order) versus the number of somatic variants. **e**, Average number of structural variations per tumour stage (INSS). Group sizes and the definition of the error bars as in figure 1b.

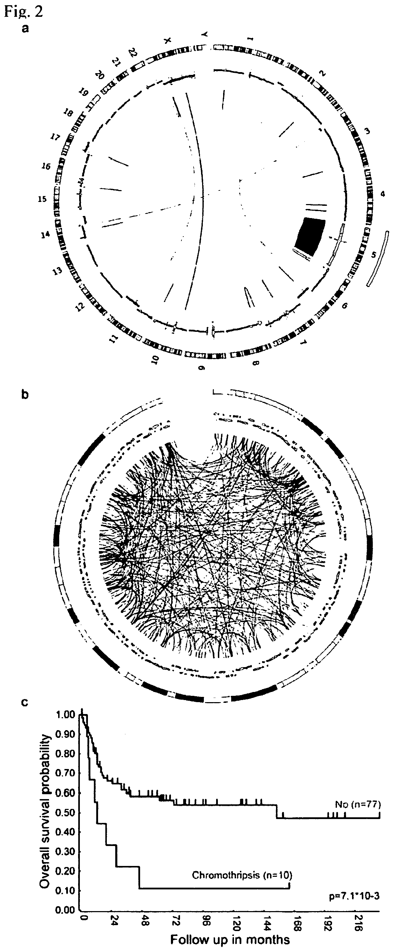

**Figure 2** **(A-C) shows that chromothripsis is frequent in neuroblastoma and is associated with a poor prognosis. a**, Circos plot showing structural variations in sample N492. The inner ring represents the copy number variations based on coverage of the tumour and lymphocyte genomes. The lines transversing the ring indicate inter- and intra-chromosomal rearrangements identified by discordant mate pairs from paired-end reads. N492 is a chromothripsis sample with an extreme amount of junctions on chromosome 5. **b**, Circos plot of the affected chromosome 5 in sample N492. **c**, Kaplan Meier curves of the overall survival for tumours with or without chromothripsis. Numbers of patients per group are shown between brackets.

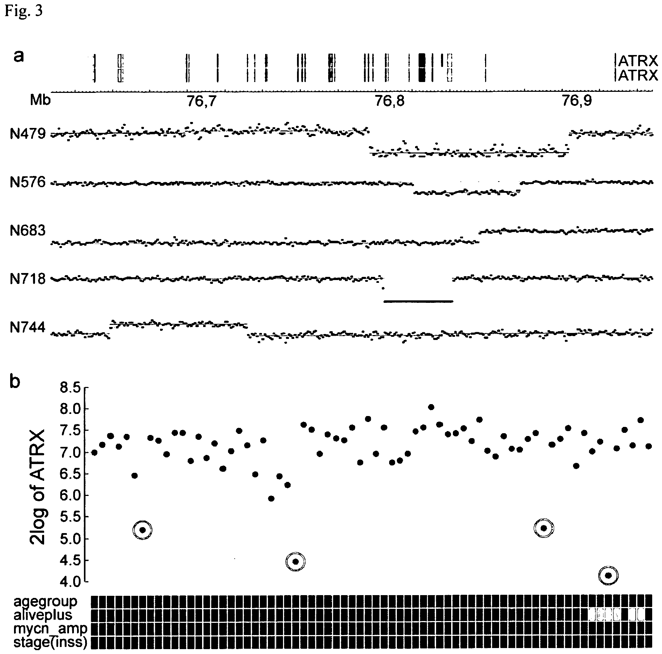

**Figure 3** **(A, B) shows that structural variations in *ATRX* result in low mRNA expression levels. a**, Coverage plots displaying the structural variations in the *ATRX* gene. The dots indicate summed coverage for bins of 1000 b.p. of the tumour genome, normalized to the coverage in corresponding normal tissue. The intron-exon structure of *ATRX* is shown in red (dark red are exons). **b**, ATRX mRNA expression of 70 tumours as measured on Affymetrix full-exon arrays. Tumours with ATRX deletions are encircled

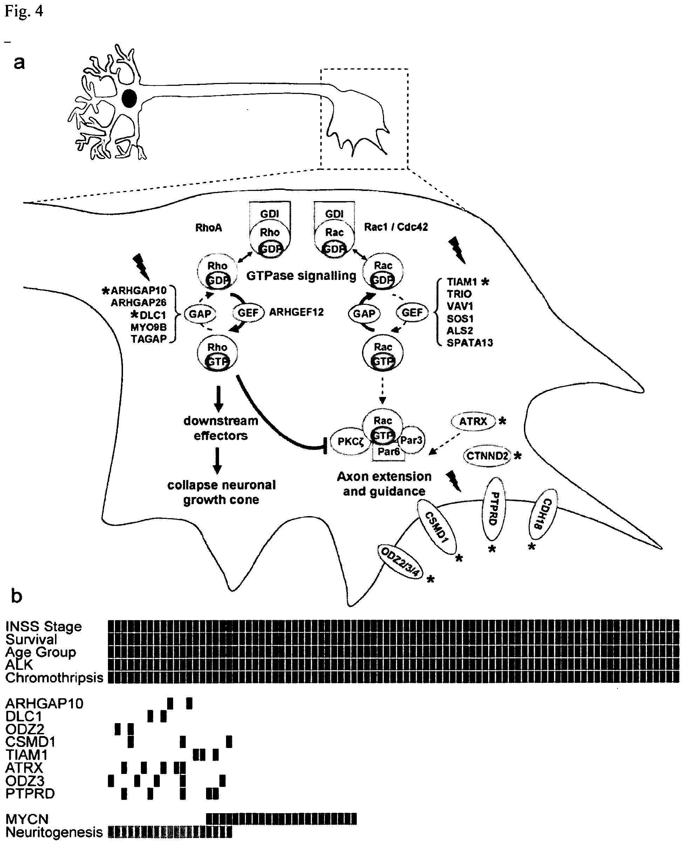

**Figure 4** **(A, B) shows Neuroblastoma with genomic defects in neuritogenesis genes cluster in high risk tumours. a**, Diagram of a neurite growth cone depicting the function of genes with genomic aberrations in neuritogenesis. The following genes in the figure were found to carry mutations in neuroblastoma: ARHGAP10, ARHGAP26, DLC1, MYO9B, TAGAP, TIAM1, TRIO, VAV1, SOS1, ALS2, SPATA13, ATRX, CTNND2, ODZ2, ODZ3, ODZ4, CSMD1, PTPRD, CDH18, ARHGEF12 (for references see table 1). Rac and Rho small GTPases cycle between an inactive GDP-bound and active GTP-bound conformation, transducing signals from a wide variety of membrane receptors. They are activated by GEFs and inactivated by GAPs. Guanine nucleotide dissociation inhibitors (GDIs) sequester GDP-bound GTPases. Genes with aberrations in more than one tumour are marked with an asterix (*). **b**, Diagram of genetic defects and clinical parameters of all 87 sequenced neuroblastoma. Each vertical lane summarizes one tumor. Patients are sorted by the presence of genomic aberrations in neuritogenesis genes (Neuritogenesis, n=19), by MYCN amplification (*MYCN,* n=23), and by INSS stage (high to low). Middle panel, amino acid changing mutations and structural variations are indicated for all genes having two or more events and that are involved in neuritogenesis (dark grey: mutated or structural variant, light grey: not affected).

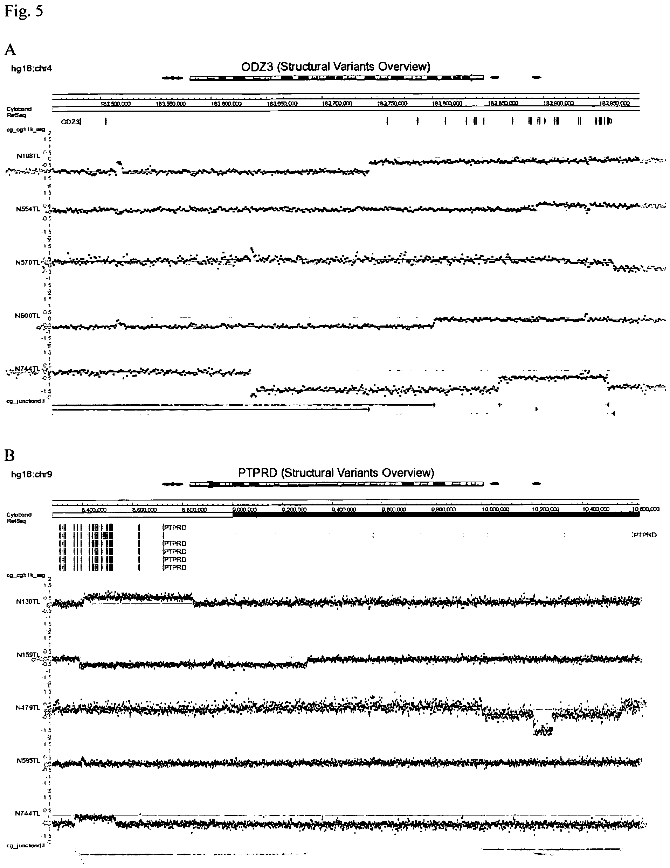

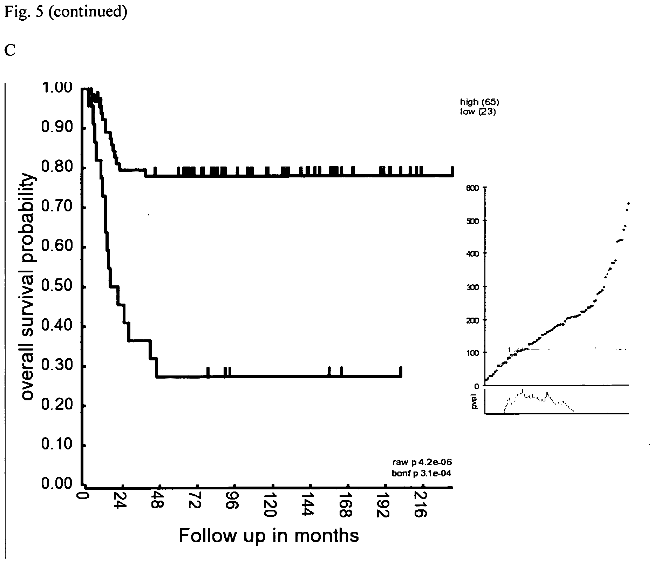

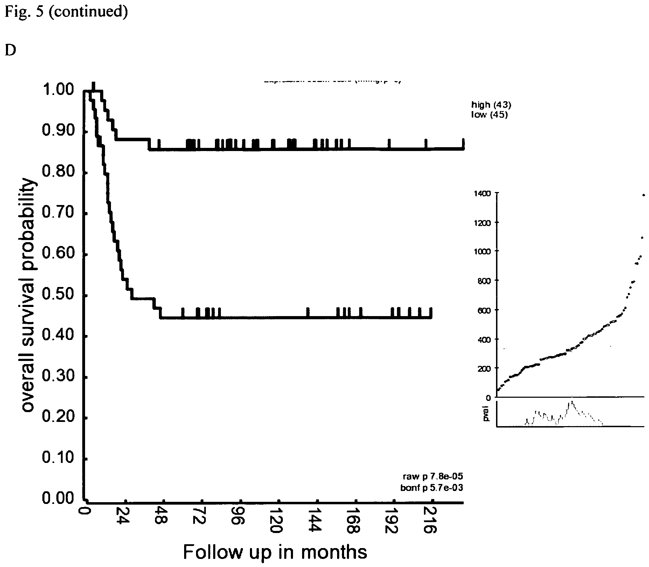

**Fig. 5** **(A, B) shows the frequent structural aberrations in PTPRD and ODZ3 and adverse patient survival:** copy number variation plots displaying structural variations in the ODZ3 (A) and PTPRD (B) genes. The dots indicate summed coverage for bins of 1000bp sections of tumour normalized to the same analysis in corresponding normal tissue. The arrow indicates a tumour with potential homozygous inactivation of ODZ3. This tumor has 3 copies of the ODZ3 region. One deletion affects two copies while the remaining copy shows a tandem duplication. C, D: Kaplan-scans for survival based on gene expression levels of ODZ3 (C) and PTPRD (D). Significance was established by logrank test ('raw p'), as well as after correction for multiple testing (Bonferoni correction) of cut-off levels for each gene ('bonfp'). Number between brackets indicates the number of patients in each group. The dot plots to the right of the Kaplan curves show the expression values for each patient, their survival status (green=alive, red=diseased). Below those, grey bars indicate -101og of the logrank pvalue, showing that the groups separation is significant in a wide range of expression cut-offs.

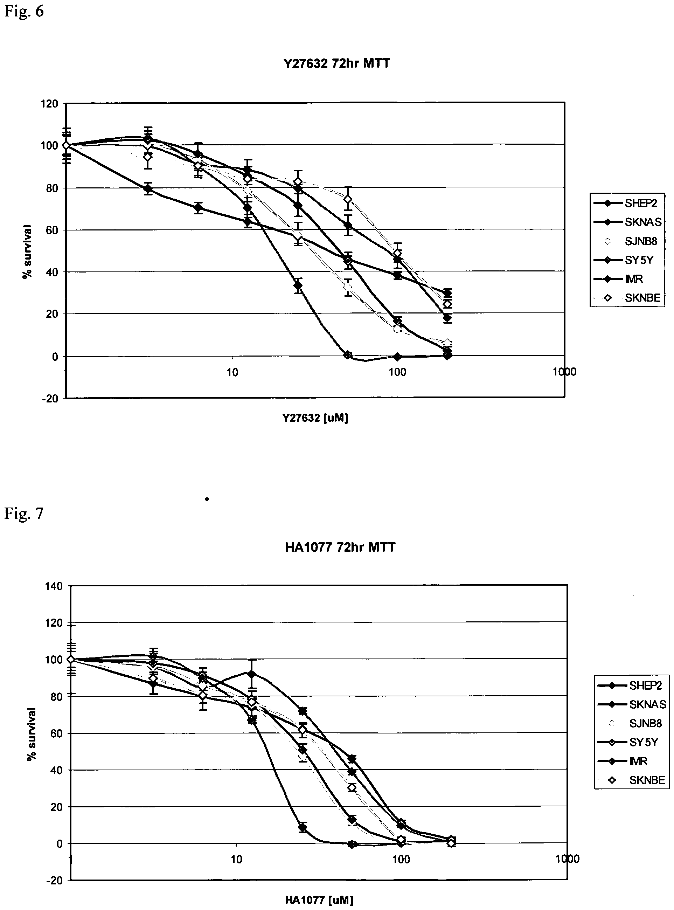

**Figure 6** **shows the curve of cell viability determined by an MTT-assay after treatment of cells with Rock-inhibitor Y27632 for 72 hours.** Cell viability is represented on the Y-axis (untreated cells at 100%) and the Y27632 concentration in micromolar is represented on the X-axis. All 6 neuroblastoma cell lines are sensitive to Y27632 and show a decrease in viability.
**Figure 7** **shows the curve of cell viability determined by an MTT-assay after treatment of cells with Rock-inhibitor HA1077 for 72 hours**. Cell viability is represented on the Y-axis (untreated cells at 100%) and the HA1077 concentration in micromolar is represented on the X-axis. All 6 neuroblastoma cell lines are sensitive to HA1077 and show a decrease in viability.

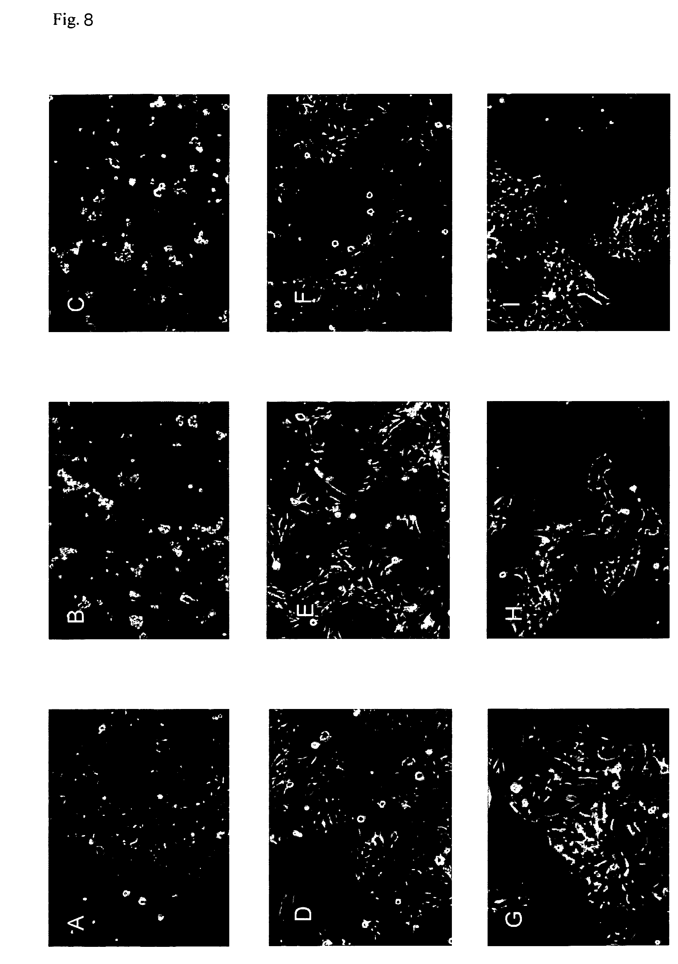

**Figure 8** **shows light-microscopy pictures of cells treated with Rock-inhibitor HA1077 or Y27632 for 65 hours**. The left panels show the normal phenotypes of the untreated neuroblastoma cell lines IMR32 (A), SJNB8 (D) and SKNBE (G). The phenotype of the cells changes when IMR32, SJNB8 and SKNBE are treated with 25µM of HA1077 (B, E and H) or Y27632 (C, F and 1) for 65 hours. They show a more neuronal-like (differentiated) phenotype; the cell bodies are smaller, more elongated and neurite outgrowths appear.

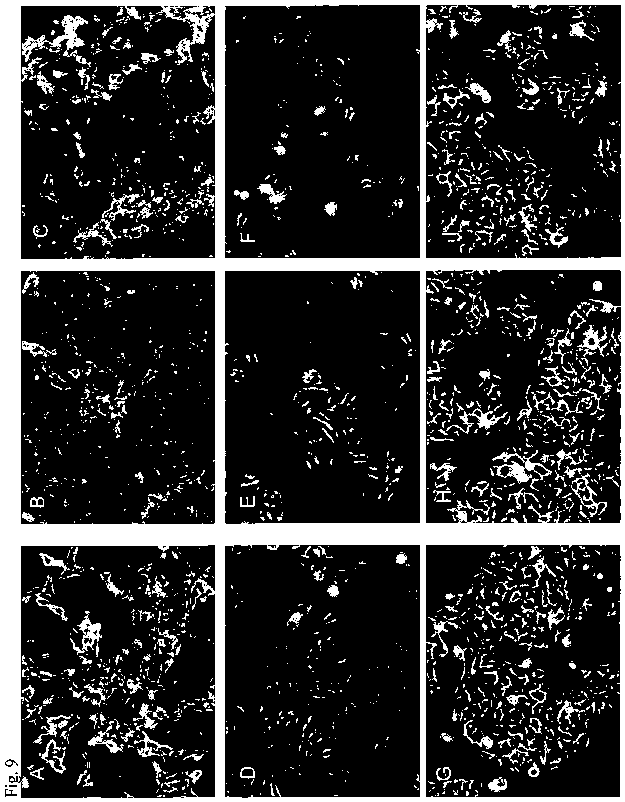

**Figure 9** **shows light-microscopy pictures of cells treated with Rock-inhibitor HA1077 or Y27632 for 65 hours.** The left panels show the normal phenotypes of the untreated neuroblastoma cell lines SY5Y (A), SHEP2 (D) and SKNAS (G). The phenotype of the cells changes when SY5Y, SHEP2 and SKNAS are treated with 25µM of HA1077 (B, E and H) or Y27632 (C, F and I) for 65 hours. They show a more neuronal-like (differentiated) phenotype; the cell bodies are smaller, more elongated and neurite outgrowths appear.

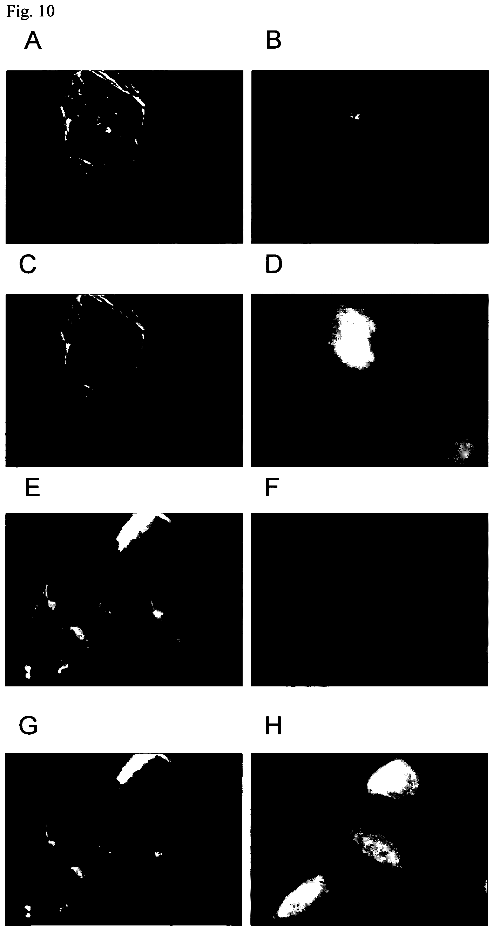

**Figure 10****: SHEP2 cells were treated with the Rock-inhibitor HA1077 (0 micromolar** **fig. 10 A-D**, **50 micromolar** **fig. 10 E-H**). **After 24 hours of treatment immunofluorescence was performed**. The cells were stained with α-Phalloidin antibody (C, G), B-tubulin antibody (D, H) and DAPI (B, F). During treatment with Rock inhibitor, the structured actin fibers (D and H) dissappear within the cells and actin is more present at the cell surface, where neurites are formed. Tubulin moves from a nuclear localization towards the cytoplasm. Fig. 10A and E show combined images of DAPI, α-Phalloidin and B-tubulin.

### DETAILED DESCRIPTION OF THE INVENTION

### Definitions

The term "ROCK" or "Rho-associated protein kinase" as used herein refers to a kinase belonging to the AGC (PKA/ PKG/PKC) family of serine-threonine kinases. It is mainly involved in regulating the shape and movement of cells by acting on the cytoskeleton. ROCK is a downstream effector protein of the small GTPase Rho. ROCKs (ROCK 1 and ROCK2) occur in mammals (human, rat, mouse, cow), zebrafish, Xenopus, invertebrates (C. elegans, Mosquito, Drosophila) and chicken. Human ROCK1 has a molecular mass of 158 kDa and is a major downstream effector of the small GTPase RhoA. Mammalian ROCK consists of a kinase domain, a coiled-coil region and a Pleckstrin homology (PH) domain, which reduces the kinase activity of ROCKs by an autoinhibitory intramolecular fold if RhoA-GTP is not present

ROCK I is also referred to as ROK [beta] or p160ROCK) and ROCK II is also referred to as ROK [alpha] or Rho kinase) (Mol. Cell. Biol., 4, 446-456 (2003).

The term "ROCK inhibitor" (abbreviated herein as "ROCKI") used herein is a compound that inhibits both or either one of the ROCKs.

The term "differentiation agent" as used herein generally encompasses any agent, such as, e.g., a chemical and/or biological substance or composition, which exerts a desirable effect on the differentiation and/or maturation of said cells. In embodiments, a differentiation agent may be a growth factor, a cytokine, an interleukin, a hormone, a protein, an enzyme, a vitamin, a metabolite, a nutrient, a small molecule, a chemical compound, a solvent, or a chemical element.

The term "immunotherapeutic agent" or as used herein includes but is not limited to anticancer immunotherapeutic agents such as antibodies to tumor antigens, or derived portions of such antibodies. In preferred embodiments, said immunotherapeutic agent is directed against GD2, preferably a monoclonal antibody. The term immunotherapeutic agent also includes any immune-system based therapeutic agent known to act against cancer, including agents that activate T-cells. T-cell activation is known to produce, among other activities, release of interleukins, which, as mentioned above, can cause localized tissue factor release.

The term "chemotherapeutic agent" as used herein is defined as a drug, toxin, compound, composition or biological entity which is used as treatment for cancer. Preferred chemotherapeutic agent are platinum compounds (cisplatin, carboplatin), alkylating agents (cyclophosphamide, ifosfamide, melphalan), topoisomerase II inhibitor (etoposide, teniposide), anthracycline antibiotics (doxorubicin) and vinca alkaloids (vincristine). In some preferred embodiments, said chemotherapeutic agents include topoisomerase I inhibitors (topotecan and irinotecan).

### Detailed description of certain embodiments of the invention

### A. RHO kinase inhibitors for the treatment of neuroblastoma

The present invention relate to Rho kinase inhibitors (ROCKIs) for the treatment of neuroblastoma in a mammal. Any ROCKI may be used. Effective ROCKI are compounds which selectively inhibit Rho Kinase, including but not limited to small compounds, antisense RNAs and specific antibodies. Many ROCKIs are available and suitable ROCKIs are those described in, for example, amide derivatives disclosed in WO 98/06433; isoquinoline sulfonyl derivatives disclosed in WO 97/23222, Nature 389, 990-994 (1997) and WO 99/64011; heterocyclic amino derivatives disclosed in WO owl/56988; indazole derivatives disclosed in WO 02/100833; and quinazoline derivatives disclosed in WO 02/076976 and WO 02/076977. Furthermore, the ROCKIs described in WO02053143, p. 7, lines 1-5, EP1163910 A1, p. 3-6, WO02076976 A2, p. 4-9, preferably the compounds described on p. 10-13 and p. 14 lines 1-3, WO02/076977A2, the compounds I-VI of p. 4-5, WO03/082808, p. 3- p. 10 (until line 14), the indazole derivates described in US7563906 B2, WO2005074643A2, p. 4-5 and preferably the specific compounds of p. 10-11, WO2008015001, pages 4-6, EP1256574 druckexemplar, claims 1-3, EP1270570 druckexemplar, claims 1-4, and EP 1 550 660. Many suitable ROCKIs are described in Tamura et al. Development of specific ROCKIs and their clinical application Biochim. Biophys. Acta 2005;1754:245-252.

Preferably, the ROCKI used in the present invention is:
- (+)-(R)-trans-4-(1-aminoethyl)-N-(4-piridyl)cyclohexanecarboxamide,
- 4[(1R)-1-aminoethyl]-N-(4-piridyl)benzamide (Fasudil),
- 1-(1-Hydroxy-5-isoquinolinesulfonyl)homopiperazine] (Hydroxyfasudil)
- 2-[4-(1H-indazol-5-yl)phenyl]-2-propanamine,
- N-(3-methoxybenzyl)-4-(4-piridyl)benzamide,
- 4-[(trans-4-aminocyclohexyl)amino]-2,5-difluorobenzamide,
- 4-[(trans-4-aminocyclohexyl)amino]-5-chloro-2-fluorobenzamide,
- 5-(hexahydro-1H-1,4-diazepin-1-ylsulfonyl)isoquinoline,
- Isoquinolinesulfonamide (K-115),
- (S)-(+)-2-methyl-1-[(4-methyl-5-isoquinoline)sulfonyl]-homopiperazine (H-1152P),
- N-(4-Pyridyl)-N'-(2,4,6-trichlorophenyl)urea,
- Y-27632,
- Y-39983, or
- ROCK I/II-specific siRNA
or a pharmaceutically acceptable salts thereof.

As intended herein, pharmaceutically acceptable salts relate to the relatively non-toxic, inorganic and organic acid addition salts, and base addition salts, of the above mentioned compounds. Exemplary salts include the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, sulphamates, malonates, salicylates, propionates, methylene-bis-b-hydroxynaphthoates, gentisates, isethionates, di-p-toluoyltartrates, methane-sulphonates, ethanesulphonates, benzenesulphonates, p-toluenesulphonates, cyclohexylsulphamates, quinateslaurylsulphonate, amine, and metal (e.g. sodium, potassium, calcium, barium, zinc, magnesium, or aluminium) salts, and the like (see, for example, Berge et al. (1977) "Pharmaceutical Salts" J. Pharm. Sc/. 66:1-19). The hydrochloride salt of Fasudil is preferred. Fasudil is obtainable from Asahi Kasei Pharma Corp (PMID: 3598899), Hydroxy fasudil is obtainable from Asahi Kasei Pharma Corp (PMID: 3598899), Y-39983 is obtainable from Novartis/Senju (PMID: 11606042) and Y27632 is obtainable from Mitsubishi Pharma (PMID: 9862451). (S)-(+)-2-Methyl-1-[(4-methyl-5-isoquinolinyl) sulfonyl]homopiperazine], N-(4-Pyridyl)-N'-(2,4,6-trichlorophenyl) urea and 3-(4-Pyridyl)-1H-indole are also available at AXXORA (UK) Ltd and other suppliers.

The following validated ROCK I/II-specific siRNA molecules may be used: ON-TARGET PLUS siRNA human ROCK I (ID: L-003536-00; Dharmacon); ON-TARGET PLUS siRNA human ROCK II (ID: L-004610-00; Dharmacon)

Highly preferred ROCKIs are compounds which are currently in clinical trials or have been tested including AR-12286 (Phase II, Aerie Pharmaceuticals), K-115 (Phase II, Kowa Pharmaceutical). Other ROCK inhibitors Y-39983/SNJ-1656/RKI-983 (Phase II, Senju and Novartis Pharmaceuticals) and INS-117548 (Phase I, Inspire Pharmaceuticals), DE-104 (Phase I/II, Santen Pharmaceuticals and Ube Industries).

AR-12286 originated from a 6-aminoisoquinoline amide series and exhibits nanomolar potency against ROCK.50 Aerie Pharmaceuticals reported that AR-12286 is more selective than a competing ROCK inhibitor Y-39983/SNJ-1656/RKI-983 (Senju and Novartis) with activity at 5 versus 25 of the off-target kinases that were tested.

K-115, an isoquinolinesulfonamide compound, is a highly selective and potent (IC50 = 31 nM) Rho-kinase inhibitor.54 It is in Phase II clinical development in patients with POAG or ocular hypertension by Kowa Pharmaceuticals (Trial IDs: JapicCTI-090708 and JapicCT1-101015).

Y-39983 (synonym RKI-983) is in Phase II clinical trials with Senju and Novartis Pharmaceuticals.

### B. Pharmaceutical composition for the treatment of neuroblastoma

Pharmaceutical compositions containing the Rho kinase inhibitor of the present invention may be manufactured by processes well known in the art, e.g., using a variety of well-known mixing, dissolving, granulating, levigating, emulsifying, encapsulating, entrapping or lyophilizing processes. The compositions may be formulated in conjunction with one or more physiologically acceptable carriers comprising excipients and auxiliaries which facilitate processing of the active compounds into preparations which can be used pharmaceutically. Proper formulation is dependent upon the route of administration chosen. Parenteral routes are preferred in many aspects of the invention.

For injection, including, without limitation, intravenous, intramuscular and subcutaneous injection, the compounds of the invention may be formulated in aqueous solutions, preferably in physiologically compatible buffers such as physiological saline buffer or polar solvents including, without limitation, a pyrrolidone or dimethylsulfoxide.

The compounds described herein may also be formulated for parenteral administration, e.g., by bolus injection or continuous infusion. Formulations for injection may be presented in unit dosage form, e.g., in ampoules or in multi-dose containers. Useful compositions include, without limitation, suspensions, solutions or emulsions in oily or aqueous vehicles, and may contain adjuncts such as suspending, stabilizing and/or dispersing agents. Pharmaceutical compositions for parenteral administration include aqueous solutions of a water soluble form, such as, without limitation, a salt (preferred) of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances that increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions. Alternatively, the active ingredient may be in powder form for constitution with a suitable vehicle, e.g., sterile, pyrogen-free water, before use.

For oral administration, the compounds can be formulated by combining the active compounds with pharmaceutically acceptable carriers well-known in the art. Such carriers enable the compounds of the invention to be formulated as tablets, pills, lozenges, dragees, capsules, liquids, gels, syrups, pastes, slurries, solutions, suspensions, concentrated solutions and suspensions for diluting in the drinking water of a patient, premixes for dilution in the feed of a patient, and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropyl-methylcellulose, sodium carboxy-methylcellulose, and/or polyvinylpyrrolidone (PVP). If desired, disintegrating agents may be added, such as cross-linked polyvinyl pyrrolidone, agar, or alginic acid. A salt such as sodium alginate may also be used.

For administration by inhalation, the compounds of the present invention can conveniently be delivered in the form of an aerosol spray using a pressurized pack or a nebulizer and a suitable propellant.

The compounds may also be formulated in rectal compositions such as suppositories or retention enemas, using, e.g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

Other delivery systems such as liposomes and emulsions can also be used.

Additionally, the compounds may be delivered using a sustained-release system, such as semi-permeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the particular compound, additional stabilization strategies may be employed.

### C. Dosages

A therapeutically effective amount refers to an amount of a compound effective to prevent, alleviate or ameliorate Neuroblastoma. Determination of a therapeutically effective amount is well within the capability of those skilled in the art, especially in light of the disclosure herein.

For any compound used for the treatment of neuroblastoma according to the invention, the therapeutically effective amount can be estimated initially from in vitro assays. Then, the dosage can be formulated for use in animal models so as to achieve a circulating concentration range that includes the effective dosage. Such information can then be used to more accurately determine dosages useful in patients.

The amount of the composition that is administered will depend upon the parent molecule included therein. Generally, the amount of medicament used in the treatment of neuroblastoma is that amount which effectively achieves the desired therapeutic result in mammals. Naturally, the dosages of the various compounds can vary somewhat depending upon the parent compound, rate of in vivo hydrolysis, molecular weight of the active compound, etc. In addition, the dosage, of course, can vary depending upon the dosage form and route of administration.

By way of example Fasudil can be administered at a dosage of from 50 mg/ day to 500 mg/day, in particular at 240 mg/day, with unit doses of from 50 mg to 500 mg, in particular 80 mg.

The treatment protocol can be based on a single dose administered once every three weeks or divided into multiple doses which are given as part of a multi-week treatment protocol. Thus, the treatment regimens can include one dose every three weeks for each treatment cycle and, alternatively one dose weekly for three weeks followed by one week off for each cycle. It is also contemplated that the treatment will be given for one or more cycles until the desired clinical result is obtained.

Those skilled in the art will determine the optimal dosing of the prodrug selected based on clinical experience and the treatment indication. Moreover, the exact formulation, route of administration and dosage can be selected by the individual physician in view of the patient's condition. The precise dose will depend on the stage and severity of the condition, and the individual characteristics of the patient being treated, as will be appreciated by one of ordinary skill in the art.

Additionally, toxicity and therapeutic efficacy of the compounds described herein can be determined by standard pharmaceutical procedures in cell cultures or experimental animals using methods well-known in the art.

Further aspects of the present invention include combining the compounds described herein with other anticancer therapies for synergistic or additive benefit.

### D. Kit

When the lesion is localized, it is generally curable. However, long-term survival for children with advanced disease older than 18 months of age is poor despite aggressive multimodal therapy (intensive chemotherapy, surgery, radiation therapy, stem cell transplant, differentiation agent isotretinoin also called 13-cis-retinoic acid, and frequently immunotherapy with anti-GD2 monoclonal antibody therapy). 13-cis-retinoic acid (isotretinoin or Accutane) and antibody therapy is preferably administered with the cytokines GM-CSF and IL-2.

With current treatments, patients with low and intermediate risk disease have an excellent prognosis with cure rates above 90% for low risk and 70%-90% for intermediate risk. In contrast, therapy for high-risk neuroblastoma the past two decades resulted in cures only about 30% of the time. The addition of antibody therapy has raised survival rates for high-risk disease significantly. In March 2009 an early analysis of a Children's Oncology Group (COG) study with 226 high-risk patients showed that two years after stem cell transplant 66% of the group randomized to received ch14.18 antibody with GM-CSF and IL-2 were alive and disease-free compared to only 46% in the group that did not receive the antibody. The randomization was stopped so all patients enrolling on the trial will receive the antibody therapy.

Chemotherapy agents used in combination have been found to be effective against neuroblastoma. Agents commonly used in induction and for stem cell transplant conditioning are platinum compounds (cisplatin, carboplatin), alkylating agents (cyclophosphamide, ifosfamide, melphalan), topoisomerase II inhibitor (etoposide), anthracycline antibiotics (doxorubicin) and vinca alkaloids (vincristine). Some newer regimens include topoisomerase I inhibitors (topotecan and irinotecan) in induction which have been found to be effective against recurrent disease.

### E. Treatment of Neuroblastoma

The present invention provides methods of treatment of neuroblastoma. For purposes of the present invention, "treatment" or "cure" shall be understood to mean inhibition, reduction, amelioration and prevention of tumor growth, tumor burden and metastasis, remission of tumor, or prevention of recurrences of tumor and/or neoplastic growths in patients after completion of treatment.

Treatment is deemed to occur when a patient achieves positive clinical results. For example, successful treatment of neuroblastoma shall be deemed to occur when at least 20% or preferably 30%, more preferably 40% or higher (i.e., 50%) decrease in tumor growth including other clinical markers contemplated by the artisan in the field is realized when compared to that observed in the absence of the treatment described herein. Other methods for determining changes in a neuroblastoma clinical status resulting from the treatment described herein include: (1) blood and urine tests for levels of catecholamine metabolites such as homovanillic acid (HVA), vanillylmandelic acid (VMA), dopamine and noreinephrine; (2) imaging tests such as X-rays, computed tomography (CT, CAT scan), magnetic resonance imaging (MRI scan), ultrasound, positron emission tomography (PET scan), MIBG scans; (3) biopsies such as tumor biopsy, bone marrow biopsy; (4) immunohistochemistry study using antibody, radioisotope, dye; and complete blood count (CBC).

In a further aspect of the present invention, the treatment described herein can be followed by retinoic acid therapy. 13-cis-retinoic acid can be given after completion of the treatment with a therapeutically effective dosis of Rho kinase inhibitor as defined herein, the pharmaceutical composition as defined herein, or the kit as defined herein. The retinoic acid therapy slows the cancer's ability to make more cancer cells, and changes how these cells look and act.

In a still further aspect, the treatment according to the method of the invention is performed concurrently or sequentially with radiation therapy. In one embodiment, radioactive iodine such as MIBG (meta-iodbenzylguanidine, radioionated with I-131 or I-123) can be provided internally and/or externally. Radiation therapies are also contemplated.

The present invention can be also performed in combination with bone marrow stem cell transplantation, and peripheral blood stem cell transplantation, and with other therapies, i.e., monoclonal antibody therapy.

In a still further aspect of the invention, the methods include treatment of neuroblastoma related to topoisomerase I-associated neuroblastoma. Other aspects of the invention include treatment of neuroblastomas which are resistant or refractory to other anticancer agents such as CPT-11, epidermal growth factor receptor antagonists (for example, Erbitux(R) cetuximab or C225) therapies and combinations thereof.

The above disclosure generally describes the present invention. Unless defined otherwise, all technical and scientific terms used herein have the same meaning as those commonly understood by one of ordinary skill in the art to which this invention belongs. A more complete understanding can be obtained by reference to the following specific examples which are provided herein for purposes of illustration only, and are not intended to limit the scope of the invention.

### EXAMPLE SECTION

### Example 1:

The inventors performed whole genome paired-end sequencing employed by Complete Genomics Inc. (Mountain View, Ca.)¹³ for 87 untreated primary neuroblastoma tumours of all stages and their corresponding lymphocyte DNAs. All samples had a minimal tumour content of 80% as determined by immunohistochemical analysis. Genomes were sequenced at an average coverage of 50 and an average fully called genome fraction of 96.6%. Compared to the HG18 reference genome the inventors obtained an average of 3,347,592 single nucleotide variants (SNVs) per genome, in accordance with reported frequencies of interpersonal variants. CGAtools was employed to compare tumour with lymphocyte genomes and provided a somatic score estimating the likelihood of mutations to be somatic (http://cgatools.sourceforge.net/docs/1.3.0/). Validation of 1,014 candidate somatic small mutations (SNVs, substitutions, insertions, deletions), including 763 SNVs established a specificity of 88% and a sensitivity of 85% at a somatic score cut-off of 0.1. SNVs above this score and all validated SNVs with lower scores were used for further analyses (total 586 genes). The sequence data identified an average of 12 somatic candidate amino-acid affecting mutations per tumour (Fig. 1a). The frequency of somatic events strongly correlated to tumour stage where stage 1, 2 and 4S tumours have very few mutations compared to stage 3 and 4 tumours (ANOVA p=7.6* 10⁻⁶; Fig. 1 b). In addition mutation frequencies were strongly correlated to overall survival (logrank p=9.8* 10⁻⁷; Fig. 1 c) and age at diagnosis (r=0.53, p=1.1* 10⁻⁷; Fig. 1 d), as was also observed in medulloblastoma¹⁴. Within high stage neuroblastoma, *MYCN* amplification status did not correlate to mutation frequency (not shown).

Only very few recurrent mutations were identified. ALK mutations were found in 6% of the tumours, in accordance with frequencies established in large neuroblastoma tumour series²⁻⁵. Three tumours carried mutations in *TIAM1,* a known regulator of cytoskeleton organisation and neuritogenesis¹⁵. In a parallel study the inventors sequenced 4 primary neuroblastoma tumours as well as cell lines derived from these tumours and their metastases. This revealed that primary tumours are already heterogeneous for mutations and that the large majority of them were passenger or late mutations (Molenaar et al., submitted). Together with the lack of recurrent mutations, our data suggest that neuroblastoma carry few early somatic tumour-driving mutations with amino-acid changing consequences.

Analysis of the paired-end clones with discordant ends can be used to identify candidate structural rearrangements, which together with sequence coverage data can identify somatic structural variants (SVs). Comparison of tumour versus lymphocyte coverage generated ultra high resolution CGH-like profiles (Fig. 3). Analysis of the frequency of structural variations per chromosome revealed 10 tumours with chromothripsis characteristics⁶. Chromothripsis is a localized shredding of a chromosomal region and subsequent random reassembly of the fragments. An extreme example of chromothripsis in chromosome 5 is shown in figure 2a and 2b. The neuroblastoma with chromothripsis were associated with a poor prognosis (logrank test p=7.1* 10⁻³; Fig. 2c). They were found in 18% of the stage 3 and 4 neuroblastoma, but not in low stage tumours (Fisher's Exact p=0.01). Accordingly, their prognostic impact is not independent of age and stage in multivariate analyses. Chromothripsis-related structural aberrations frequently affected genes involved in neuroblastoma pathogenesis and were associated with amplification of *MYCN* or *CDK4* and LOH of 1p. In one tumour, chromothripsis resulted in amplification and very strong over-expression of *MYC (c-Myc).* Chromosome 5 had undergone chromothripsis in 3 tumours, but no clear tumourigenic target on this chromosome was identified. To identify genetic defects that allowed chromothripsis and subsequent survival of the cell, the inventors searched for defects in DNA damage response pathways in tumours with chromothripsis. The most extreme case of chromothripsis (N492, Fig. 2a and 2b) showed an inactivating deletion in *FANCM* and another chromothripsis tumour sample (N576) had a missense mutation in *FAN1* predicted to be damaging by the polyphen2 program¹⁶. These findings might suggest involvement of inactivating events in the Fanconi anaemia signalling pathway to allow chromothripsis¹⁷.

Full genome paired-end sequencing allowed us to identify structural variants specifically perturbing single genes. The inventors detected a total of 451 genes harbouring structural variants (306 genes without the events on chromothripsis chromosomes). The structural variants often consisted of deletions of one or a few exons, inversions or translocations deleting part of a gene. One tumour showed an intrachromosomal rearrangement activating *FOXR1* transcription, which the inventors recently identified as a recurrent but rare event in neuroblastoma¹⁸. Similar to the findings for amino-acid changing mutations, there was a strong relation between the frequency of structural variations and the tumour stage (one way ANOVA p=0.03; Fig. 1e), which extends the well established relationship between tumour stage and structural chromosomal defects in neuroblastoma¹⁰⁻¹². Breakpoints identifying deletions were supported by changes in coverage plots. Most of the structural variants affected only one allele of a gene. This suggests that the tumour driving mechanism of these defects is haploinsufficiency possibly combined with epigenetic attenuation of the non-affected allele. On average, genes with structural variants resulting in loss of coverage indeed showed a reduced expression in tumours with these defects, as compared to tumours with normal alleles. As an additional validation, the inventors generated SNP-arrays of 52 of the sequenced tumours. Although the SNP data have a much lower resolution than the sequence coverage plots, they supported the deletions and gains of sufficient size. This is especially evident on plots of chromothripsis samples.

To identify relevant genes and pathways that contribute to neuroblastoma pathogenesis, the inventors generated one list of all genes with amino-acid changing mutations (n=586), mutations in splice junctions (n=37) and structural variations (n=451). The total of 1,041 genes with alterations were analysed by two approaches. Firstly, the inventors analyzed the most frequently affected genes. Four genes belonged to the *MYCN* amplicons (*MYCN, MYCNOS, DDX1* and *NBAS*) and except for *MYCN* probably play no role in pathogenesis. Three genes, *PTPRD, ODZ3* and *ATRX,* showed structural variants in five tumours each (Fig. 3a and fig. 5) and 61 genes showed alterations in two to four tumours. A conservative randomization taking the length of all genes and the structure of our data set into account, showed that the chance of finding three genes affected in five or more tumors is <2.11* 10⁻⁴. This strongly indicates that at least the defects in *PTPRD, ATRX* and *ODZ3* did not accumulate due to e.g. the genomic length of the genes, but that they were selected for during the process of tumorigenesis.

The X-chromosome encoded *ATRX* gene was affected by structural variants in five tumors (Fig. 3a). In two male patients this resulted in complete inactivation of the gene. Frequent *ATRX* defects were recently found in pancreatic neuroendocrine tumours¹⁹. ATRX is a chromatin remodelling protein involved in exchange of H3.3 in GC-rich repeats and mutations of this gene are associated with X-linked mental retardation²⁰. Exon mRNA profiles of part of the sequenced series showed that the three included samples with *ATRX* structural variations had the lowest *ATRX* mRNA expression of all samples and showed a specific collapse of the signal in the deleted regions, illustrating the inactivating nature of the *ATRX* defects (Fig. 3b).

*ODZ3* and *PTPRD* were also hit by structural variations in 5 tumors each (Figure 5). One tumor showed homozygous inactivation of *ODZ3* (see legends of Supplementary fig. 5). In addition, *ODZ2* and *ODZ4,* two highly homologous members of the conserved ODZ family, were together affected three times. *PTPRD* and ODZ genes encode transmembrane receptors expressed in the developing nervous system and localizing to axons and axonal growth cones²¹. Targeted silencing of ODZ homologues in drosophila, C. elegans and mouse caused severe axon guidance defects⁹. Over-expression of *ODZ2* in neuroblastoma cells enhanced neuritogenesis²². PTPRD is a member of the LAR sub-family of receptor protein tyrosine phosphatases. Transgenic mouse models strongly implicate the LAR sub-family receptors in neuritogenesis⁸. *PTPRD* defects in neuroblastoma were previously reported by Stallings and co-workers²³. Low expression of *ODZ3* and of *PTPRD* as assessed by mRNA profiling were both associated with a poor prognosis (logrank p=3.1* 10⁻⁴ and p=5.7*10⁻³, respectively; Figure 5). Interestingly, *CSMD1,* which showed structural variants in three tumors, also is a transmembrane protein expressed on nerve growth cones⁷. As the frequencies of *PTPRD* and *ODZ3* defects exclude that they were found by chance, the inventors hypothesize that the function of these genes and *of ODZ2, ODZ4* and *CSMD1* in neuronal growth cones might hold a clue to their function in neuroblastoma pathogenesis.

The second analysis that the inventors performed for the list for 1,041 affected genes was a Gene Ontology (GO) study to identify enrichment of genes with defects in specific molecular processes. The GO-category 'regulation of GTPase activity' was the most significantly enriched group (Bonferoni corrected for multiple testing: p= 6.7*10⁻⁴). This finding urged the inventors to further investigate GTPase regulating genes in the list. *TIAM1* was mutated in three tumours. It functions as a guanine nucleotide exchange factor (GEF) for the small GTPase Rac and is, together with Rac, central to regulation of cellular polarity and neuritogenesis^{24,25}. The W1285S* mutation creates a premature stop-codon in in the C- terminal Plekstrin homology domain required for Rac activation, while the other mutations were predicted to be damaging by polyphen2 analysis¹⁶. Rac is activated by GEFs and inactivated by GTPase Activating Proteins (GAPs)²⁶ (Fig 4). The inventors identified a total of 8 alterations in 6 GEFs specific for Rac (including *TL4M1*), but none in GAPs specific for Rac (Table 1). While activation of Rac 1 stimulates neuritogenesis, activation of its small GTPase antagonist RhoA promotes axon retraction and growth cone collapse (Fig. 4a)¹⁵. Strikingly, the inventors detected 7 alterations in 5 GAPs for RhoA, while only one GEF specific for RhoA *(ARHGEF12)* showed a translocation with unknown functional consequences (Fig. 4a, Table 1). The bias for inactivation of GAPs for Rho and GEFs for Rac is highly significant (One sided Fischer exact test: p<0.0007). This suggests that alterations in GTPase-regulating genes specifically function to activate Rho or inactivate Rac, which both tip the balance in Rac/Rho signalling towards inhibition of neuritogenesis. Of note, transgenic mice with *ATRX* mutations causing mental retardation in humans showed abnormal dendritic spine formation with increased *TIAM1* phosphorylation and Rac1 signalling²⁷.

The inventors conclude that alterations with significant frequencies *(PTPRD* and ODZ genes) affect transmembrane receptors that function in neuronal growth cone guidance and maintenance. In addition GO analysis of the 1041 genes showed significant enrichment of GTPase regulating genes. Alterations in GEFs for Rac and GAPs for Rho significantly deviate from a random distribution, implicating inhibition of Rac 1 and activation of RhoA in impairing neuritogenesis in neuroblastoma (Fig 4a).

From these findings the inventors hypothesize that defects in neuritogenesis-regulating genes form an important category of tumour-driving events in neuroblastoma. For a preliminary analysis of tumors with these defects, the inventors selected the genes with recurrent defects in tumors that function in neuronal growth cones (*PTPRD, ODZ3, ODZ2, CSMD1*) or regulation of these processes through Rac/Rho signalling (*TL4M1, DLC1, ARHGAP10, ATRX*)*.* The 19 tumours with defects in these genes were almost all stage 3 and 4 tumours diagnosed above 1.5 year of age with an aggressive clinical course. Only few of them showed amplification of *MYCN* (Fig. 4b). Consistent with their occurrence in high stage neuroblastoma, defects in neuritogenesis genes did not show an independent prognostic power in multivariate analysis with the clinical parameters age and stage.

Here the inventors report the first whole genome sequence study of a comprehensive series of neuroblastoma including both low and high stage tumours. Low stage neuroblastoma lacked recurrent gene alterations (mutations and structural variations), raising the question whether they are primarily driven by chromosomal imbalances and the consequent gene dosage effects. Tumours with defects in genes functioning in neuritogenesis or growth cone guidance mostly are aggressive high stage tumours without *MYCN* amplification. Interestingly, there is indication that MYCN also inhibits neuritogenesis in neuroblastoma. MYCN down-regulates the mRNA expression of the chromosome 1p36 gene *CDC42,* which resulted in inhibition of neuritogenesis of neuroblastoma cells²⁸. CDC42 is a small GTPase protein with a function similar to Rac1. Rac1 and CDC42 both regulate the Par3/Par6 complex involved in cell polarization and growth cone development and which has been shown to drive neuroblastoma cell differentiation²⁵. In light of our data demonstrating genomic alterations in Rho/Rac signalling, it is tempting to postulate that the Par3/Par6 complex is a recurrent target for inactivation in neuroblastoma. Intriguingly, the block in neuritogenesis of neuroblastoma is probably not absolute. Retinoic acid (RA) can induce neuronal outgrowth in many neuroblastoma cell lines, which all are derived from high stage tumours. RA is also used in long term neuroblastoma treatment protocols to prevent recurrences²⁹. It is currently unknown which tumours will clinically respond to RA therapy. Deletions of *NF1* were previously implicated in RA resistance of neuroblastoma cell lines³⁰. The identification of genomic alterations in a range of genes mediating neuritogenesis now allows investigation of therapeutic modalities to surmount these defects.

### Methods summary

All neuroblastoma samples were derived from primary tumors of untreated patients. Tumor material was obtained during surgery and a portion was immediately frozen in liquid nitrogen. The inventors used leukocytes derived from peripheral blood for isolation of constitutional DNA. High-molecular-weight DNA was extracted from tumor tissue and leukocytes using standard procedures. Fifteen microgram of DNA was subjected to paired end whole genome sequencing according to the Complete Genomics (Mountain View CA) technology. Initial data analyses were performed using the CGAtools v1.3.0 package (http://cgatools.sourceforge.net/docs/1.3.0/) and for subsequent analysis and figure preparation the inventors used the R2 bioinformatic platform (http://r2.amc.nl) and PERL scripts. More details on mutation analysis, coverage analysis, analysis of structural variants and the Circos plots is given in the supplemental information. Gene expression was assayed on Affymetrix EXON ST 1.0 GeneChips. SNP genotyping microarray analysis details are described in the supplementary information. Mutation validations were performed using Sanger sequencing (supplemental information). Statistical analysis (Gene Ontology enrichment using the cytoscape BINGO plugin, DAVID functional ontology cluster analysis and analysis for gene length enrichment) are described in the supplemental information.

### Example 2:

The inventors performed experiments with ROCK-inhibitors on a panel of 6 neuroblastoma cell lines (SKNBE, IMR, SJNB8, SHEP2, SKNAS, SY5Y), which together are a good representation of the heterogeneity of neuroblastoma.

To determine the sensitivity of the cell lines for the ROCK-inhibitors Y27632 and HA 1077 MTT assays were done. All 6 neuroblastoma cell lines are sensitive to Y27632 (Figure 6) and HA 1077 (Figure 7) and show a decrease in viability. The IC50 (concentration drug needed for 50% cell viability reduction) of Y27632 determined by dose efficacy curves varied from 18 to 90 µM. The IC50 of HA 1077 determined by dose efficacy curves varied from 15 to 45 µM.

To study the phenotypical changes of the cell lines after treatment with ROCK-inhibitors Y27632 and HA 1077, the inventors first made images of the cells using a normal light-microscope. The neuroblastoma cells changed toward a more neuronal-like (differentiated) phenotype (Figure 8 and 9). The cell bodies are smaller, more elongated and neurite outgrowths appear.

Secondly, the inventors performed immunofluorescence analyses of cells. After treatment with HA 1077, the structured actin fibres (green) disappear within the SHEP2 cells and actin is more present at the cell surface, where neurites are formed (Figure 10). Tubulin moves from a nuclear localization towards the cytoplasm. This suggests a rigorous change in the organization of the cytoskeleton, which is required for the transformation toward a more differentiated phenotype.

### MTT-assay

Cells were plated in a 10-30% confluence in a 96-well plate and treated after 24 hours with Y27632 (=Y27632 dihydrochloride; Tocris; Cat.nr. 1254) and HA1077 (=Fasudil hydrochloride; Tocris; Cat.nr. 0541) in a concentration ranging from 3 to 200 µM. 72 hours after treatment, 10 µl of Thyazolyl blue tetrazolium bromide (MTT, Sigma M2128) was added. After 4 hours of incubation 100 µl of 10% SDS, 0.01 M HCI was added to stop the reaction. The absorbance was measured at 570 nm and 720 nm using a platereader (biotech). The IC50 (concentration drug needed for 50% cell viability reduction) was calculated using concentration vector curves.

### Immunofluorescence

Cells were grown on glass slides in 6-well plates. Cells were fixed with 4% paraformaldehyde in PBS 24 hours after treatment. The cells were permeabilized with 0.1% Triton X-100/PBS and incubated with Superblock (Thermo Fisher Scientific TA-125-UB). The inventors used mouse anti-β tubulin (1:200, Sigma-aldrich T8660) as a primary antibody and goat anti-mouse (Alexa, A11029) for secondary antibody. Phalloidine-FITC (1:100, Sigma-aldrich P5282) was used for the actin staining. Slides were stained with DAPI (1:1000) in vectashield (Vector laboratories).

### REFERENCES

¹ J. M. Maris, "Recent advances in neuroblastoma," N. Engl. J. Med. 362(23), 2202 (2010).
² Y. P. Mosse, et al., "Identification of ALK as a major familial neuroblastoma predisposition gene," Nature 455(7215), 930 (2008).
³ R. E. George, et al., "Activating mutations in ALK provide a therapeutic target in neuroblastoma," Nature 455(7215), 975 (2008).
⁴ Y. Chen, et al., "Oncogenic mutations of ALK kinase in neuroblastoma," Nature 455(7215), 971 (2008).
⁵ I. Janoueix-Lerosey, et al., "Somatic and germline activating mutations of the ALK kinase receptor in neuroblastoma," Nature 455(7215), 967 (2008).
⁶ P. J. Stephens, et al., "Massive genomic rearrangement acquired in a single catastrophic event during cancer development," Cell 144(1), 27 (2011).
⁷ D. M. Kraus, et al., "CSMD1 is a novel multiple domain complement-regulatory protein highly expressed in the central nervous system and epithelial tissues," J. Immunol. 176(7), 4419 (2006).
⁸ Q. L. Sun, et al., "Growth cone steering by receptor tyrosine phosphatase delta defines a distinct class of guidance cue," Mol. Cell Neurosci. 16(5), 686 (2000).
⁹ L. Zheng et al., "Drosophila Ten-m and filamin affect motor neuron growth cone guidance," PLoS. One. 6(8), e22956 (2011).
¹⁰ Q. R. Chen, et al., "cDNA array-CGH profiling identifies genomic alterations specific to stage and MYCN-amplification in neuroblastoma," BMC. Genomics 5, 70 (2004).
¹¹ J. Vandesompele, et al., "Multicentre analysis of patterns of DNA gains and losses in 204 neuroblastoma tumors: how many genetic subgroups are there?," Med. Pediatr. Oncol. 36(1), 5 (2001).
¹² I. Janoueix-Lerosey, et al., "Overall genomic pattern is a predictor of outcome in neuroblastoma," J. Clin. Oncol. 27(7), 1026 (2009).
¹³ R. Drmanac, et al., "Human genome sequencing using unchained base reads on self-assembling DNA nanoarrays," Science 327(5961), 78 (2010).
¹⁴ D. W. Parsons, et al., "The genetic landscape of the childhood cancer medulloblastoma," Science 331(6016), 435 (2011).
¹⁵ F. N. Leeuwen, et al., "The guanine nucleotide exchange factor Tiam1 affects neuronal morphology; opposing roles for the small GTPases Rac and Rho," J. Cell Biol. 139(3), 797 (1997).
¹⁶ I. A. Adzhubei, et al., "A method and server for predicting damaging missense mutations," Nat. Methods 7(4), 248 (2010).
¹⁷ Y. Kee and A. D. D'Andrea, "Expanded roles of the Fanconi anemia pathway in preserving genomic stability," Genes Dev. 24(16), 1680 (2010).
¹⁸ E. E. Santo, et al., "Oncogenic activation of FOXR1 by 11q23 intrachromosomal deletion-fusions in neuroblastoma," Oncogene (2011).
¹⁹ Y. Jiao, et al., "DAXX/ATRX, MEN1, and mTOR pathway genes are frequently altered in pancreatic neuroendocrine tumors," Science 331(6021), 1199 (2011).
²⁰ M. J. Law, et al., "ATR-X syndrome protein targets tandem repeats and influences allele-specific expression in a size-dependent manner," Cell 143(3), 367 (2010).
²¹ C. O. Arregui, J. Balsamo, and J. Lilien, "Regulation of signaling by protein-tyrosine phosphatases: potential roles in the nervous system," Neurochem. Res. 25(1), 95 (2000).
²² B. P. Rubin, et al., "Teneurins: a novel family of neuronal cell surface proteins in vertebrates, homologous to the Drosophila pair-rule gene product Ten-m," Dev. Biol. 216(1), 195 (1999).
²³ R. L. Stallings, et al., "High-resolution analysis of chromosomal breakpoints and genomic instability identifies PTPRD as a candidate tumor suppressor gene in neuroblastoma," Cancer Res. 66(7), 3673 (2006).
²⁴ N. Matsuo, et al., "Roles of STEF/Tiam1, guanine nucleotide exchange factors for Racl, in regulation of growth cone morphology," Mol. Cell Neurosci. 24(1), 69 (2003).
²⁵ T. Nishimura, et al., "PAR-6-PAR-3 mediates Cdc42-induced Rac activation through the Rac GEFs STEF/Tiaml," Nat. Cell Biol. 7(3), 270 (2005).
²⁶ D. Bar-Sagi and A. Hall, "Ras and Rho GTPases: a family reunion," Cell 103(2), 227 (2000).
²⁷ N. Shioda, et al., "Aberrant calcium/calmodulin-dependent protein kinase II (CaMKII) activity is associated with abnormal dendritic spine morphology in the ATRX mutant mouse brain," J. Neurosci. 31(1), 346 (2011).
²⁸ L. J. Valentijn, et al., "Inhibition of a new differentiation pathway in neuroblastoma by copy number defects of N-myc, Cdc42, and nm23 genes," Cancer Res. 65(8), 3136 (2005).
²⁹ K. K. Matthay, et al., "Long-term results for children with high-risk neuroblastoma treated on a randomized trial of myeloablative therapy followed by 13-cis-retinoic acid: a children's oncology group study," J. Clin. Oncol. 27(7), 1007 (2009).
³⁰ M. Holzel, et al., "NF1 is a tumor suppressor in neuroblastoma that determines retinoic acid response and disease outcome," Cell 142(2), 218 (2010).

## Claims

1. A Rho kinase inhibitor for the treatment of neuroblastoma.

2. Rho kinase inhibitor according to claim 1, causing differentiation of neuroblastoma cells.

3. Rho kinase inhibitor according to claim 1, selected from the group consisting of:
- (+)-(R)-trans-4-(1-aminoethyl)-N-(4-piridyl)cyclohexanecarboxamide,
- 4[(1R)-1-aminoethyl]-N-(4-piridyl)benzamide (Fasudil),
- 1-(1-Hydroxy-5-isoquinolinesulfonyl)homopiperazine] (Hydroxyfasudil)
- 2-[4-(1H-indazol-5-yl)phenyl]-2-propanamine,
- N-(3-methoxybenzyl)-4-(4-piridyl)benzamide,
- 4-[(trans-4-aminocyclohexyl)amino]-2,5-difluorobenzamide,
- 4-[(trans-4-aminocyclohexyl)amino]-5-chloro-2-fluorobenzamide,
- 5-(hexahydro-1H-1,4-diazepin-l-ylsulfonyl)isoquinoline,
- Isoquinolinesulfonamide (K-115),
- (S)-(+)-2-methyl-1-[(4-methyl-5-isoquinoline)sulfonyl]-homopiperazine (H-1152P),
- N-(4-Pyridyl)-N'-(2,4,6-trichlorophenyl)urea,
- Y-27632,
- Y-39983, and
- ROCK siRNA
or a pharmaceutically acceptable acid addition salt thereof.

4. A pharmaceutical composition for the treatment of neuroblastoma, comprising as active compound a therapeutically effective amount of the Rho kinase inhibitor as defined in any of claims 1-3 and a pharmaceutically acceptable carrier.

5. Pharmaceutical composition according to claim 4, further comprising a further medicament having anti-neuroblastoma activity

6. A kit comprising the pharmaceutical composition as defined in claim 4 and a further medicament having anti-neuroblastoma activity.

7. Pharmaceutical composition according to claim 5 or kit according to claim 6, wherein said further medicament is selected from the group consisting of:
a) a differentiation agent;
b) an immunotherapeutic agent directed against neuroblastoma cells;
c) a chemotherapy agent
for simultaneous, separate or sequential use for the treatment of neuroblastoma.

8. A method for treating a patient suffering from neuroblastoma which comprises administering a therapeutically effective dosis of Rho kinase inhibitor as defined in claim 1, 2 or 3, the pharmaceutical composition as defined in 4, 5 or 7, or the kit as defined in claim 6 or 7.
